Europäisches Patentamt

European Patent Office

Office européen des brevets

(11) Numéro de publication: **0 286 761 B1**

# FASCICULE DE BREVET EUROPEEN

(45) Date de publication de fascicule du brevet: **14.04.93**  (51) Int. Cl.⁵: **C12P 19/06**, C08B 37/00, E21B 43/25

(21) Numéro de dépôt: **87400847.7**

(22) Date de dépôt: **14.04.87**

(54) **Procédé enzymatique de traitement de gommes xanthanes en vue d'ameliorer la filtrabilté de leurs solutions aqueuses.**

(43) Date de publication de la demande:
**19.10.88 Bulletin 88/42**

(45) Mention de la délivrance du brevet:
**14.04.93 Bulletin 93/15**

(84) Etats contractants désignés:
**DE GB NL SE**

(56) Documents cités:
EP-A- 0 039 962    FR-A- 2 264 077
FR-A- 2 551 087    GB-A- 2 065 688
GB-A- 2 099 008    US-A- 4 165 257

(73) Titulaire: **INSTITUT FRANCAIS DU PETROLE**
**4, Avenue de Bois Préau**
**F-92502 Rueil-Malmaison(FR)**

(72) Inventeur: **Ballerini, Daniel**
**103, rue du Pontel**
**F-78100 St Germain en Laye(FR)**
Inventeur: **Benoit, Yves**
**2 Mail Renaissance**
**F-95120 Ermont(FR)**
Inventeur: **Monot, Frédéric**
**9 Parc du Belloy**
**F-78600 Le Mesnil Le Roi(FR)**

## Description

L'objet de la présente invention est de décrire une amélioration au procédé du brevet US-A-4.431.734 au nom de la demanderesse.

Dans ce brevet on a décrit un procédé enzymatique de traitement de gommes xanthanes en vue d'améliorer la filtrabilité et l'injectivité des solutions aqueuses de gommes xanthanes lors de leur injection et de leur circulation à travers des formations pétrolifères en vue d'améliorer la récupération d'huile brute. Ce procédé comprend un traitement approprié à l'aide de deux systèmes enzymatiques, le premier de type polysaccharase en pH acide ou sensiblement neutre et le deuxième de type protéase en pH basique, neutre ou acide, et permet d'améliorer l'injectivité et l'écoulement des solutions de xanthanes à travers les formations pétrolifères sans perte des propriétés intrinsèques du polysaccharide et en particulier de son caractère épaississant.

Selon ce brevet le traitement enzymatique à l'aide d'un enzyme de type polysaccharase et d'un enzyme de type protéase peut être effectué soit simultanément à un pH compatible avec une activité suffisante des deux types d'enzymes soit consécutivement avec un pH convenable pour le type d'enzyme choisi dans chaque étape. Les meilleurs résultats sont obtenus quand on opère en deux étapes successives, la première étape étant effectuée avec la polysaccharase, puis la deuxième étape étant effectuée avec la protéase.

Les extraits enzymatiques appelés polysaccharases, obtenus habituellement par culture aérobie de champignons appartenant à la classe des Basidiomycètes, ou de champignons appartenant par exemple aux genres Aspergillus, Fusarium, Myrothecium, Penicillium, Polyporus, Rhizopus, Sclerotinia, Sporotrichum et Trichoderma sont utilisables dans le procédé de ce brevet. Ces enzymes susceptibles d'hydrolyser les polysaccharides sont habituellement commercialisés sous le nom de cellulases et sont utilisés dans le procédé selon ce brevet dans des conditions de pH, de température et de concentrations salines telles que les caractéristiques de la gomme xanthane elle-même ne soient pas sensiblement affectées.

La deuxième catégorie d'extraits enzymatiques utilisables de manière complémentaire à la précédente catégorie dans le procédé selon ce brevet est constituée par la classe des protéases bactériennes. Ces protéases sont en général produites par des microorganismes du genre Bacillus tels que B. Subtilis, B. licheniformis, B. amyloliquefaciens et B. pumilis ou encore du genre Streptomyces tels que S. fradiae, S. griseus et S. rectis. La source de l'enzyme n'est toutefois pas critique. Ces protéases ont une activité optimum selon le cas à des valeurs de pH légèrement acide, neutre ou basique et sont alors dénommées respectivement protéases acides, neutres ou alcalines.

Le traitement enzymatique selon ce procédé a lieu de préférence pendant une période d'incubation dont la durée totale est de 0,5 à 60 heures et de préférence de 4 à 48 heures, à des températures allant de la température ambiante (25°C environ) jusqu'à environ 65°C et de préférence de 40 à 60°C. Les temps de traitement courts seront de préférence associés aux températures élevées et inversement. Les traitements enzymatiques sont effectués en milieu aqueux de concentration en sels dissous de métaux alcalins et/ou alcalino-terreux d'au moins $10^{-2}$ équivalent/litre, de préférence au moins $10^{-1}$ équivalent/litre. L'effet de synergie relatif obtenu dans ce procédé est toutefois d'autant plus important que la salinité de l'eau de traitement est plus forte. Un aspect particulier du procédé selon ce brevet réside dans le fait que l'activité de synergie des deux préparations enzymatiques s'obtient également en présence d'ions bivalents, par exemple $Ca^{++}$ ou $Mg^{++}$ et en particulier d'eau de gisement.

Un premier objet de la présente invention est de fournir une méthode améliorée de clarification de solutions aqueuses de gommes xanthanes, dans laquelle le pouvoir épaississant de ces gommes est conservé. Un autre objet de l'invention consiste en l'amélioration de la clarification du jus brut de la fermentation ainsi que des dispersions aqueuses de gommes xanthanes disponibles sous forme de poudre. Un autre objet de l'invention consiste dans l'élimination des débris cellulaires insolubles provenant du processus de fermentation de ces gommes xanthanes. Un autre objet de l'invention consiste dans l'amélioration de l'injectivité des solutions de gommes xanthanes pour l'utilisation en récupération assistée du pétrole. Encore un autre objet de l'invention réside dans l'élimination des microgels et donc dans l'amélioration des propriétés d'écoulement des solutions de gomme xanthane à l'intérieur d'une formation pétrolifère à une certaine distance du puits injecteur. Finalement, un autre objet de l'invention réside dans l'utilisation de compositions solides permettant d'améliorer la limpidité, l'injectivité et l'écoulement de solutions de gommes xanthanes.

L'objet de la présente invention est de proposer l'emploi d'autres préparations ou extraits enzymatiques pour la première catégorie d'extraits enzymatiques employés dans le procédé suivant le brevet US-A-4.431.734.

Selon la présente invention on emploie, au lieu d'une préparation enzymatique de type polysaccharase, encore appelée cellulase, dont l'activité enzymatique principale est une activité cellulolytique, un extrait enzymatique dont l'activité principale est une activité polygalacturonase. Ainsi selon la présente invention on emploie deux extraits enzymatiques de types différents, un extrait enzymatique dit extrait PG défini ci-après et un extrait enzymatique dit extrait P défini ci-après, dans des conditions compatibles avec l'activité desdits extraits enzymatiques.

Les extraits enzymatiques que l'on emploie dans la présente invention contiennent généralement plusieurs autres activités, il est essentiel que, pour l'extrait PG, l'activité polygalacturonase soit l'activité principale, les autres activités par exemple cellulase, protéase acide, xylanase etc... n'étant que des activités secondaires. Les extraits PG employés ne possèdent généralement que peu, et de préférence, sensiblement pas d'activité cellulase. On emploiera de préférence un extrait enzymatique dont l'activité polygalacturonase est de type endo.

Par activité principale, on entend l'activité enzymatique généralement largement prépondérante parmi les nombreuses activités que possède généralement un extrait enzymatique. On favorise l'excrétion de cette activité par le choix des conditions et du milieu de culture et de la souche de microorganisme producteur dans le cas où l'extrait enzymatique est d'origine microbienne ou fongique. On peut encore procéder à une séparation des enzymes conduisant à un enrichissement et une purification orientés vers l'obtention d'une activité donnée.

Dans le cas des enzymes utilisés dans le brevet US-A-4.431.734 la polysaccharase obtenue par culture de Basidiomycete genre Poria qui est préconisée dans ce brevet, a une activité principale de type cellulase d'environ 50 000 unités Carboxyméthylcellulase (CMCase) par gramme de préparation, soit d'environ 250 unités par mg de protéines.

L'activité CMCase qui mesure l'activité cellulase d'un extrait enzymatique correspond au nombre de micromoles de glucose libéré à partir de carboxyméthylcellulose, l'essai étant conduit 30 minutes à pH 4,6 et 37°C. Afin de pouvoir comparer les activités enzymatiques de préparations sous forme pulvérulente et sous forme liquide, il est préférable d'utiliser les activités spécifiques (par mg de protéines).

L'activité polygalacturonase d'un extrait enzymatique correspond au nombre de micromoles d'acide galacturonique libéré à partir d'acide polygalacturonique, l'essai étant conduit 30 minutes à pH 4,0 et 40°C.

L'activité polygalacturonase de la polysaccharase du brevet américain précité, commercialisée sous le nom de cellulase, obtenue à partir de Basidiomycete du genre Poria, est d'environ 5 unités par mg de protéines.

Les activités enzymatiques de type polygalacturonase sont habituellement produites par cultures de bactéries du genre Erwinia, Pseudomonas, de levures du genre Kluyveromyces ou de champignons du genre Aspergillus, Rhizopus, Fusarium, Rhizoctonia, Penicillium, Sclerotinia et Verticillium.

A partir d'une souche de microorganisme, par exemple Aspergillus niger, il est possible d'obtenir un extrait enzymatique dont l'activité enzymatique principale est, soit de type cellulase (polysaccharase), soit de type polygalacturonase. Le choix de la source de carbone entrant dans la composition du milieu de culture est important pour orienter le microorganisme vers la production principale d'une activité enzymatique déterminée. Ainsi pour l'obtention de polygalacturonase comme activité principale, il est souvent nécessaire de prendre un hydrate de carbone contenant de la pectine (pectine commerciale, son de blé, pulpes de betteraves...). Le milieu peut comprendre en outre des sources de sels minéraux et d'azote. Après culture par exemple à 30°C pendant quelques jours, le milieu est débarassé des cellules par filtration ou centrifugation et peut être utilisé comme extrait enzymatique ayant une activité principale polygalacturonase. Il est ensuite possible d'enrichir l'extrait en protéines par exemple par ultrafiltration ou par précipitation des protéines à l'aide de solvants (par exemple acétone, éthanol) ou de sels (par exemple sulfate d'ammonium).

Selon la présente invention on utilise des extraits enzymatiques qui ne possèdent que peu d'activité cellulase. Des exemples de préparations industrielles sont en particulier les extraits enzymatiques appelés pectinases obtenus à partir d'une culture d'un champignon du genre Aspergillus et plus particulièrement à partir d'Aspergillus niger.

L'activité protéase des extraits enzymatiques de type PG, mesurée en unité ANSON par gramme d'extrait enzymatique comme décrit ci-après, est habituellement inférieure à 0,05 unité et de préférence inférieur à 0,01 et très avantageusement inférieure à 0,005. Si l'on mesure les activités enzymatiques spécifiques des extraits en unités par milligramme de protéine et si l'on désigne par "C" l'activité cellulase et par "PG" l'activité polygalacturonase de ces extraits, on emploie de préférence dans la présente invention des extraits enzymatiques dont le rapport C/PG est inférieur à 1 et de préférence inférieur à 0,5 et d'une façon la plus préférée inférieur à 0,1.

Ainsi selon la présente invention on améliore la filtrabilité de solutions aqueuses de gommes xanthanes en effectuant un traitement des dites solutions par un extrait enzymatique dont l'activité principale est une activité polygalacturonase (extrait PG) et par un extrait enzymatique ayant comme activité principale une activité protéase (cet extrait sera dénommé extrait P) dans des conditions compatibles avec l'activité des dits extraits.

L'activité protéase peut être déterminée par la méthode de KUNITZ. Dans ce cas, 1 unité protéase correspond à la quantité d'extrait enzymatique libérant une quantité de substances non précipitables au TCA (acide trichloroacétique) ayant une densité optique à 550 nm équivalente à la densité optique donnée par 0,4 g de tyrosine avec le réactif de FOLIN. La température de l'essai est de 37°C et le temps de réaction de 20 minutes. Dans le cas des protéases alcalines ou neutres, le substrat est de la caséine à 1 % et dans le cas des protéases acides, le substrat est de la sérumalbumine bovine à 1 %. Les pH de mesure des activités protéases alcalines, neutres et acides sont respectivement de 10, 7 et 3.

D'autres unités protéases sont utilisées comme l'unité ANSON qui est la quantité d'enzyme qui dans des conditions standards (25°C ; pH 7,5 ; 10 minutes) digère l'hémoglobine à une vitesse initiale telle qu'elle libère par minute une quantité de produits solubles dans le TCA, qui donne la même coloration avec le réactif phénol qu'un milliéquivalent de tyrosine.

Ainsi, par exemple, l'ALCALASE 0,6 L (Marque commerciale de Novo Industrie A/S) est une préparation liquide obtenue par fermentation de Bacillus licheniformis qui contient comme activité principale (protéase) 0,6 unité ANSON par gramme et aucune autre activité enzymatique appréciable.

D'une manière générale, les extraits enzymatiques issus de bactéries du genre Bacillus ayant comme activité principale l'activité protéase ne contiennent pratiquement pas d'activités polygalacturonase ou cellulase parmi leurs activités secondaires.

Habituellement les extraits enzymatiques ayant pour activité principale l'activité protéase que l'on utilise dans la présente invention ont des activités polygalacturonase et cellulase exprimées en unités par milligramme de protéines respectivement inférieures à 5 et de préférences inférieures à 1 et d'une façon souvent avantageuse inférieures à 0,5.

Les extraits enzymatiques ayant pour activité principale l'activité protéase ont une activité mesurée en unité ANSON par gramme d'extrait enzymatique habituellement d'au moins 0,1 unité et de préférence de 0,2 à 10 unités.

Un des autres intérêts de la présente invention est de n'incorporer à la solution de polysaccharides que de faibles quantités de protéines en choisissant une préparation enzymatique particulièrement active pour l'élimination des agents colmatants présents au sein des solutions de polymère. Lorsqu'on utilise une préparation enzymatique telle que celle du brevet US-A-4.431.734 ayant comme activité principale l'activité cellulase et contenant parmi les activités secondaires l'activité polygalacturonase, il est nécessaire d'ajouter des quantités d'enzymes (donc de protéines) relativement importantes. Par exemple dans le cas de la cellulase obtenue à partir de Basidiomycete genre Poria, on introduit, selon l'exemple 1 de ce brevet environ 30 % en poids d'enzyme par rapport au polysaccharide. Or il est apparu que les protéines peuvent jouer un rôle d'agent colmatant dans les solutions de polysaccharides si elles sont présentes en quantité trop importante. Par conséquent, il est fortement préférable d'ajouter le moins de protéines possibles dans le cas d'un traitement visant à améliorer la filtrabilité des solutions de polysaccharides. C'est pourquoi il est judicieux d'utiliser une préparation enzymatique agissant principalement au niveau des agrégats, ce qui permet de diminuer le taux de matière active (donc de protéines) à incorporer pour avoir un traitement efficace. On a découvert de façon surprenante que des extraits enzymatiques dits extraits PG permettent d'améliorer fortement la filtrabilité des solutions de xanthane en particulier lorsque l'on associe le traitement par un extrait enzymatique ayant la dite activité à un traitement par un extrait enzymatique dit extrait P de la classe des protéases bactériennes. On peut alors en employant ces deux traitements clarifier des solutions de polysaccharide avec des quantités de protéines peu élevées, nettement inférieures à celles utilisées dans le procédé selon le brevet US-A-4.431.734.

Le traitement enzymatique de la présente invention peut être réalisé soit en la présence simultanée d'un extrait PG et d'un extrait enzymatique dit extrait P à une valeur de pH compatible avec une activité suffisante des deux types d'activités, soit consécutivement à l'aide d'abord d'un extrait enzymatique de l'un des deux types ci-dessus en pH convenable pour le type choisi, puis d'un extrait enzymatique de l'autre type en pH convenable pour cet autre type, par exemple l'extrait pG, en pH acide suivi de l'extrait P en pH légèrement acide, neutre ou basique selon le type d'extrait P, ou l'inverse. Les meilleurs résultats sont obtenus quand on opère en deux étapes successives d'abord avec l'extrait PG puis avec l'extrait P.

Le traitement enzymatique de l'invention est effectué en milieu aqueux de concentration en sels dissous de métaux alcalins et/ou alcalino-terreux d'au moins $10^{-2}$ équivalent/litre, de préférence au moins $10^{-1}$ équivalent/litre. L'effet de synergie relatif obtenu est relativement insensible à la salinité. Bien que des

EP 0 286 761 B1

concentrations salines supérieures à environ 1 équivalent/litre puissent être employées, on préfère généralement utiliser des concentrations comprises entre au moins $10^{-2}$ équivalent/litre et au plus environ 1 équivalent/litre. Un aspect particulier de la présente invention réside dans le fait que l'activité de synergie des deux types d'enzymes s'obtient également en présence d'ions bivalents, par exemple $Ca^{++}$ ou $Mg^{++}$, et en particulier d'eau de gisement.

Le traitement enzymatique simultané ou consécutif de la présente invention a lieu de préférence pendant une période d'incubation dont la durée totale est de 0,5 à 60 heures et de préférence de 4 à 48 h, à des températures allant d'environ 15°C jusqu'à environ 70°C, de préférence de 20 à 60°C. Les temps de traitement courts seront de préférence associés aux températures élevées et inversement. Si l'on choisit d'utiliser le traitement enzymatique aux températures les plus élevées, le temps optimum pourra être relativement court par exemple 2 - 24 h à 50°C, 1 - 12 heures à 60°C. Les températures préférées vont de 20 à 60°C et ne devront de préférence pas excéder 70°C, température au-delà de laquelle les extraits enzymatiques sont susceptibles de se désactiver de manière notable.

Les extraits PG sont utilisés, dans le procédé conforme à la présente invention, dans des conditions de pH (3 < pH < 7), de température (15 - 70°C) et de concentration saline (> $10^{-2}$ équivalent/litre) indiquées ci-dessus, telles que les caractéristiques de la gomme xanthane elle-même ne soient pas sensiblement affectées.

L'autre catégorie d'extraits enzymatiques utilisée selon l'invention est constituée par des extraits de la classe des protéases bactériennes. Ces extraits P sont habituellement produits par des microorganismes du genre Bacillus tels que B. subtilis, B. licheniformis, B. amyloliquefaciens et B. pumilis ou encore du genre Streptomyces tels que S. fradiae, S. griseus et S. rectis. La source de l'extrait n'est toutefois pas critique. Ces extraits P ont une activité optimum selon le cas à des valeurs de pH légèrement acide, neutre ou basique et sont alors dénommés respectivement extraits P acides, neutres ou alcalins. Naturellement, dans le cas d'un traitement simultané à l'aide d'un extrait PG et d'un extrait P, on choisira des espèces enzymatiques dont les domaines d'activité, en ce qui concerne le pH, se recouvrent.

Bien que le traitement de synergie de la présente invention s'applique essentiellement aux dispersions dans l'eau salée de gommes xanthanes sous forme de poudre, il va de soi qu'il peut également s'appliquer aux moûts de fermentation. Bien plus, les gommes xanthanes isolées à partir des moûts de fermentation ainsi traités ne nécessitent plus aucun traitement enzymatique ultérieur et la filtrabilité de leurs solutions aqueuses se trouve considérablement améliorée. Les techniques d'isolement à l'état de poudre d'une gomme xanthane à partir de moût de fermentation sont par ailleurs bien connues et consistent par exemple en une précipitation à l'aide d'un alcool miscible au jus de fermentation ou encore en un procédé de séchage par lyophilisation ou par évaporation du solvant.

Le procédé de synergie de la présente inventin, utilisant un traitement simultané ou consécutif par un extrait PG et par un extrait P permet, en premier lieu, d'obtenir une dégradation des débris cellulaires et bactériens solides en suspension dans les solutions de gomme xanthane en les transformant en composés hydrosolubles de sorte que l'on obtienne finalement une solution limpide. De plus, et cela est plus étonnant, ce traitement de synergie permet d'éliminer les microgels translucides, responsables du colmatage des formations pétrolifères à une certaine distance du puits injecteur. Dans toute cette opération de clarification et d'élimination des microgels, le pouvoir épaississant de la gomme xanthane est conservé et les solutions limpides obtenues peuvent ensuite, après simple dilution et sans traitement de filtration ultérieur, être injectées dans les formations pétrolifères. L'injectivité et les propriétés d'écoulement des dites solutions à travers ces formations se trouvent nettement améliorées par rapport aux traitements des extraits enzymatiques pris isolément comme cela peut être aisément démontré par les tests correspondants à travers des filtres calibrés.

En vue de réaliser le traitement de synergie simultané selon le procédé de la présente invention, on peut procéder comme suit au départ de dispersions dans une phase aqueuse ayant la salinité requise ci-dessus de gommes xanthanes en poudre ou de dilutions par la même phase aqueuse de moûts bruts de fermentation : on ajuste si nécessaire le pH de la dite solution à la valeur de pH correspondant à l'activité optimale des deux types d'extraits enzymatiques et l'on ajoute ces deux extraits enzymatiques. On maintient la température à 15 - 70°C pendant des temps variables pour obtenir l'amélioration de la filtrabilité décrite ci-dessus. On réajuste si nécessaire le pH de la solution à la valeur du pH d'utilisation et après dilution à la concentration et à la viscosité désirée, la solution ainsi traitée est prête à l'emploi.

Dans le cas où l'on souhaite effectuer le traitement enzymatique de la présente invention en deux étapes, on peut opérer comme suit : on amène si nécessaire le pH de la solution de gomme xanthane à une valeur inférieure à 7 et supérieure à 3, avantageusement un pH de 3 à 6, à l'aide par exemple d'acide chlorhydrique, d'acide acétique ou d'acide sulfurique. On ajoute l'extrait PG et on maintient la température à 15 - 70°C pendant le temps nécessaire défini plus haut, après quoi on ramène le pH de la solution à

5

l'aide d'une base par exemple la soude ou la potasse à des valeurs de pH supérieures à 6 et inférieures à 12, avantageusement un pH de 6,5 à 9. Après addition de l'extrait P, on maintient à nouveau la température à 15 - 70°C pendant le temps nécessaire, défini plus haut. Après avoir réajusté le pH de la solution à la valeur du pH d'utilisation et après dilution à la concentration et à la viscosité désirée, la solution ainsi traitée est prête à l'emploi.

Une variante du traitement enzymatique en deux étapes consiste à ajuster tout d'abord le pH de la solution à une valeur comprise de préférence entre 6,5 et 9 et à réaliser le traitement enzymatique par l'extrait P avant de réajuster le pH à des valeurs légèrement acides, de préférence entre 3 et 6 et d'effectuer le traitement enzymatique par l'extrait PG.

Lors du traitement enzymatique de la présente invention, la proportion de gomme xanthane est, par exemple, de 0,01 à 4 % et de préférence de 0,04 à 1,5 % en poids, par rapport à l'eau et la proportion de chaque extrait enzymatique est, par exemple de 0,01 à 10 % en poids de protéines, de préférence 0,025 à 5 % en poids de protéines par rapport au poids de xanthane, ces proportions n'étant pas limitatives. La quantité minimale d'extraits enzymatiques à utiliser est évidemment fonction de la quantité de facteur actif (donc d'activité polygalacturonase et protéase) dans les préparations enzymatiques choisies.

Selon un aspect additionnel de la présente invention, des formulations solides contenant la gomme xanthane et les deux types d'extraits enzymatiques peuvent être ajoutées directement à l'eau de gisement, éliminant de ce fait toute nécessité d'addition séparée des extraits enzymatiques à la solution de gomme xanthane, ceci dans l'optique du traitement enzymatique simultané. Ces compositions solides sont d'un intérêt particulier lorsque la clarification enzymatique doit être réalisée, par exemple, sur le site même d'une opération de récupération assistée. La réaction enzymatique se fera ainsi au fur et à mesure de la solubilisation du polysaccharide et, si la température et le pH de l'eau de dissolution sont choisis convenablement, le traitement enzymatique ne rallongera pas la durée usuelle de préparation de la solution de gomme xanthane injectée. Il est possible d'obtenir ainsi directement une solution limpide, ayant la viscosité souhaitée et pouvant être utilisée directement sans aucun traitement complémentaire et en particulier de filtration et qui possède des propriétés d'injectivité et de filtrabilité nettement améliorées pour l'utilisation dans des opérations de récupération assistée.

Une telle composition solide peut renfermer, par exemple, de 10 à 100 000 et de préférence de 20 à 40 000 parties en poids de gomme xanthane par partie en poids des protéines du mélange d'extraits enzymatiques.

Les exemples suivant illustrent l'invention; ils ne doivent en aucune manière être considérés comme limitatifs.

EXEMPLE 1 (comparatif)

Cet exemple décrit un traitement enzymatique faisant intervenir une polysaccharase produite par Basidiomycete genre Poria présentant l'activité cellulase comme activité principale (25 000 Unités CMCase/l soit 250 U CMCase/mg de protéine) et une faible activité polygalacturonase (500 Unités polygalacturonase/l soit 5 U/mg protéine) parmi ses nombreuse activités secondaires.

On utilise une solution à 10 g/l de polysaccharide en poudre Rhodopol 23 (Rhône-Poulenc Industries - France) préparée dans de l'eau contenant 1 g/l de NaCl et 0,4 g/l d'azide de sodium en tant qu'agent bactériostatique. Après agitation pendant quelques heures, le pH de la solution est ajusté à 4,5 et la température est portée à 50°C. On ajoute alors 500 mg/l d'une préparation enzymatique de polysaccharase obtenue à partir du Basidiomycete genre Poria qu'on laisse agir pendant 16 heures. La quantité d'enzyme introduite équivaut à l'incorporation de 100 mg/l de protéines (1 % en poids de protéines par rapport au xanthane). Le pH est ensuite ajusté à 9,0 avec de la soude 1N et 500 mg/l d'Alcalase (marque commerciale de Novo Industri A/S) issue d'une culture de Bacillus licheniformis ayant une activité protéase de 0,6 unité ANSON par gramme sont ajoutés. Cet extrait enzymatique contient une activité protéase alcaline comme activité principale et pratiquement pas d'activité polygalacturonase, ni d'activité cellulase; ces activités sont inférieures a $10^{-2}$ unité par milligramme de protéine. Le traitement est arrêté après 6 heures d'action à 50°C de cet extrait enzymatique.

Les échantillons prélevés avant addition d'enzyme, après action de la polysaccharase et après action de l'Alcalase sont soumis au test de filtrabilité rapide décrit dans le brevet principal afin d'évaluer l'efficacité du traitement enzymatique. Ce test consiste à faire passer les solutions obtenues, après dilution à la concentration en xanthane de 0,4 g/l à l'aide d'eau à 1 g/l de NaCl et 0,4 g/l de $NaN_3$ et après que leur pH ait été ajusté à 7, à travers un filtre Millipore de 0,8 um (0 = 47 mm) sous une pression constante de 10 kPa. On enregistre le volume cumulé de filtrat au cours du temps de filtration. Les résultats figurent dans le tableau 1 ci-après.

TABLEAU 1

| N° | Solution | Volume cumulé de filtrat en 20 minutes |
|---|---|---|
| 1 | Avant traitement | 30 ml |
| 2 | Après 16 heures d'action de la polysaccharase | 160 ml |
| 3 | Après 22 heures d'action de la polysaccharase | 200 ml |
| 4 | Après 16 h (polysaccharase) puis 6 h (Alcalase) | 670 ml |

Ces résultats montrent l'efficacité du traitement combiné polysaccharase-Alcalase Novo sur la filtrabilité des solutions de xanthane et l'important effet de synergie entre les deux préparations enzymatiques. La viscosité relative des solutions n'est pratiquement pas affectée par le traitement.

EXEMPLE 2 (comparatif)

Le traitement décrit dans cet exemple a été réalisé dans des conditions rigoureusement identiques à celles du traitement n° 3 de l'exemple 1 exception faite de la quantité de polysaccharase ajoutée qui est de 125 mg/l au lieu de 500 mg/l dans l'exemple précédent (soit 0,25 % de poids de protéines par rapport au poids de xanthane). Les activités cellulase et polygalacturonase introduites sont respectivement de 6250 unités CMCase/l et de 125 unités Polygalacturonase/l alors qu'elles étaient de 25 000 unités CMCase/l et 500 U polygalacturonase/l dans l'exemple 1.

Dans ce cas l'efficacité du traitement combiné polysaccharase-Alcalase Novo est moindre puisque le volume cumulé de filtrat après action combinée de la polysaccharase et de l'Alcalase Novo est de 300 ml en 20 minutes (au lieu de 670 ml dans l'exemple 1).

EXEMPLE 3 (comparatif)

Cet exemple est destiné à montrer l'action d'une enzyme contenant comme précédemment l'activité cellulase comme activité principale, mais dont l'activité polygalacturonase est encore plus faible que celle utilisée dans l'exemple 1.

Le traitement a été effectué dans des conditions similaires à celles de l'essai n° 4 de l'exemple 1: même solution initiale, action identique de l'Alcalase Novo (toutefois 2 durées ont été testées : 2 h et 6 h), test de filtrabilité rapide.

L'enzyme utilisée en premier est une préparation enzymatique (sous forme d'extrait lyophilisé) produite par Trichoderma reesei et qui présente une forte activité cellulase (200 U/mg protéine) et une très faible activité polygalacturonase (1 U/mg protéine). La teneur en protéines de cet extrait lyophilisé est d'environ 100% et on ajoute cet extrait, en proportion de 100 mg/l de protéines, à la solution à 10 g/l de Rhodopol 23 (identique à celle de l'exemple 1) ramenée à pH 4,8 et 50°C qui constituent les conditions optimales d'action de l'enzyme. Avant ajout de l'extrait P (Alcalase Novo) on laisse agir la cellulase pendant 16 heures.

7

Les résultats des tests de filtrabilité rapides effectués figurent dans le tableau 2.

TABLEAU 2

| N° | Solution | Volume cumulé de filtrat en 20 minutes |
|---|---|---|
| 1 | Avant traitement | 30 ml |
| 2 | Après 16 heures (cellulase) | 120 ml |
| 3 | Après 16 h (cellulase) + 2 h (Alcalase) | 310 ml |
| 4 | Après 16 h (cellulase) + 6 h (Alcalase) | 310 ml |

La viscosité relative des solutions n'est pratiquement pas modifiée par l'action de ces préparations enzymatiques.

La filtrabilité finale est inférieure à celle obtenue dans l'exemple 1. Cette préparation enzymatique dont l'activité polygalacturonase est faible ne convient pas bien à l'élimination des agents colmatants présents dans la solution de polysaccharide. Cet exemple montre en particulier que l'activité cellulase n'est pas l'activité principale responsable de l'amélioration de la filtrabilité.

EXEMPLE 4

Dans cet exemple, on va utiliser une préparation enzymatique obtenue à partir d'une culture d'Aspergillus niger. Cet extrait dit extrait PG a comme activité principale l'activité polygalacturonase.

On effectue le traitement dans des conditions similaires à celles des précédents exemples, à savoir: préparation d'une solution de Rhodopol 23 à 10 g/l dans de l'eau à 1 g/l de NaCl et 0,4 g/l de NaN$_3$, action de l'extrait PG obtenu à partir d'Aspergillus niger à pH 4,0, 40°C pendant 16 heures suivie de l'action de l'Alcalase Novo de Bacillus licheniformis à pH 9,0 et 50°C pendant 2 h ou 6 h.

L'extrait PG est ajouté de manière à introduire 100 mg/l de protéines. Cette enzyme contient peu de CMCase, environ 1 unité par mg de protéine et environ 60 unités polygalacturonase par mg de protéine qui constitue l'activité principale, l'activité protéase est inférieure à 0,01 unité ANSON par gramme d'enzyme.

Les solutions prélevées sont diluées à 0,4 g/l de polymère et soumises au test de filtrabilité rapide. Les résultats figurent dans le tableau 3 et les viscosités relatives ne changent pratiquement pas au cours du traitement.

TABLEAU 3

| N° | Solution | Volume cumulé de filtrat en 20 minutes |
|----|----------|----------------------------------------|
| 1 | Avant traitement | 30 ml |
| 2 | Après 16 h (extrait PG) | 210 ml |
| 3 | Après 18 h (extrait PG) | 220 ml |
| 4 | Après 22 h (extrait PG) | 230 ml |
| 5 | Après 16 h (extrait PG) + 2 h (Alcalase) | 750 ml |
| 6 | Après 16 h (extrait PG) + 6 h (Alcalase) | 820 ml |

Les résultats montrent que la première enzyme est tout à fait adaptée à l'élimination des agents colmatants de la solution de polysaccharide et que l'effet de synergie avec l'Alcalase est remarquable. Par conséquent, le remplacement d'une enzyme présentant l'activité cellulase comme activité principale et l'activité polygalacturonase comme activité secondaire par une enzyme présentant peu d'activité cellulase et l'activité polygalacturonase comme activité principale est tout à fait conseillé puisque l'amélioration de la filtrabilité des solutions de xanthane y est meilleure.

EXEMPLE 5 (comparatif)

Le traitement décrit dans cet exemple a été réalisé dans des conditions identiques à celles de l'essai n° 6 de l'exemple 4. La préparation enzymatique utilisée est également issue d'une culture d'Aspergillus niger mais son activité spécifique polygalacturonase est de 0,8 unité par mg de protéine et son activité spécifique CMCase est de l'ordre de 100 unités par mg de protéine. La quantité de protéine introduite pour le traitement du xanthane est de 100 mg par litre de solution. La filtrabilité finale de la solution est de 200 ml en 20 minutes. Ainsi, alors qu'il provient de la culture du même microorganisme que celui de l'exemple 4, cet extrait enzymatique est peu efficace pour l'amélioration de la filtrabilité des solutions de polysaccharides.

EXEMPLE 6

Cet exemple est destiné à montrer l'efficacité d'une préparation enzymatique ayant une très forte activité polygalacturonase du fait d'une purification partielle.

Les conditions du traitement sont analogues à celles de l'exemple 4, exception faite du premier extrait enzymatique utilisé. Celui-ci est un extrait PG partiellement purifié issu d'une culture d'Aspergillus niger. Son activité polygalacturonase est d'environ 120 unités par mg de protéine et son activité CMCase est de l'ordre de 10 unités/mg protéine. Cet extrait enzymatique est incorporé à un taux de 5,6 mg protéine/l (au lieu de 100 mg/l dans les exemples précédents). Dans ce cas le rapport en poids protéines de l'extrait PG/xanthane n'est que de 0,056 %. Par rapport à l'exemple 1, le taux de CMCase introduit est environ 450 fois plus faible alors que le taux de polygalacturonase est du même ordre (670 unités/l au lieu de 500). On laisse agir cet extrait PG purifié à pH 4 et 40°C pendant 16 heures avant l'ajout d'Alcalase Novo. Les prélèvements sont soumis au test de filtrabilité rapide. Les résultats figurent dans le tableau 4.

TABLEAU 4

| Solution | Volume cumulé de filtrat en 20 minutes |
|---|---|
| Avant traitement | 30 ml |
| Après 16 h d'action de l'extrait PG purifié | 130 ml |
| Après 22 h d'action de l'extrait PG purifié | 150 ml |
| Après 16 h (extrait PG purifié) + 6 h (Alcalase) | 640 ml |

On a en outre vérifié que la viscosité relative est pratiquement constante tout au long du traitement enzymatique.

Cet exemple montre qu'en utilisant un extrait PG partiellement purifié, on peut obtenir une très bonne amélioration de la filtrabilité des solutions de xanthane avec une quantité d'enzyme, donc de protéines, très faible. La quantité de cellulase incorporée avec l'extrait PG partiellement purifié est negligeable, ce qui prouve que l'activité cellulolytique n'est pas indispensable à la clarification des solutions de polysaccharide.

Cet exemple montre aussi qu'il existe une forte synergie entre l'action de l'extrait PG et celle de l'extrait P (protéase alcaline: Alcalase Novo), ce qui permet d'obtenir un résultat pratiquement équivalent à celui obtenu dans l'exemple comparatif 1 ci-dessus en employant un taux de protéine nettement plus faible (environ 18 fois moindre) ce qui est particulièrement intéressant et permet d'éviter tout risque de colmatage dû à une quantité trop importante de protéines.

Les solutions aqueuses de gomme xanthane rendues limpides par le traitement enzymatique de la présente invention peuvent être utilisées directement, éventuellement après dilution à la concentration désirée, sans aucun traitement ultérieur, en tant que fluide de balayage des formations pétrolifères.

## Revendications

1. Procédé de traitement d'une gomme xanthane en vue d'améliorer la filtrabilité de ses solutions aqueuses comprenant un traitement enzymatique d'une solution aqueuse de gomme xanthane présentant une concentration en sels dissous de métaux alcalins et/ou alcalino-terreux d'au moins $10^{-2}$ équivalents/ litre, caractérisé en ce que le traitement enzymatique est effectué au moyen de deux extraits enzymatiques de types différents, un extrait enzymatique dit extrait PG dont l'activité principale est une activité polygalacturonase et un extrait enzymatique dit extrait P dont l'activité principale est une activité protéase, dans des conditions compatibles avec l'activité desdits extraits enzymatiques.

2. Procédé selon la revendication 1 dans lequel les deux extraits enzymatiques de types différents sont utilisés simultanément dans des conditions permettant à ces extraits des deux types d'être simultanément actifs.

3. Procédé selon la revendication 1 dans lequel le traitement enzymatique est effectué en deux étapes successives, d'abord avec un extrait enzymatique du premier type, puis avec un extrait enzymatique de l'autre type, les conditions étant choisies à chaque étape de manière à permettre au type d'extrait choisi d'être actif au cours de ladite étape.

4. Procédé selon la revendication 3 dans lequel la première étape comprend le traitement par l'extrait PG et la deuxième étape le traitement par l'extrait P.

5. Procédé selon la revendication 4 dans lequel la première étape est effectuée avec un extrait PG à un pH de 3 à 7 et la seconde étape avec un extrait P à un pH de 6 à 12, la durée totale de ces deux étapes étant de 0,5 à 60 heures et la température étant de 15 à 70°C.

6. Procédé selon l'une des revendications 1 à 5 dans lequel l'extrait PG est un extrait obtenu à partir d'une culture d'un champignon appartenant au genre Aspergillus et l'extrait P est obtenu à partir d'une culture d'un microorganisme de type Bacillus.

7. Procédé selon l'une des revendications 1 à 6 dans lequel l'extrait PG est un extrait obtenu à partir d'une culture d'Aspergillus niger.

8. Procédé selon l'une quelconque des revendications 1 à 7, caractérisé en ce que l'extrait PG possède une activité principale polygalacturonase et une activité secondaire de type cellulase (C) telle que le rapport des activités C/PG soit inférieur à 1 et une activité protéase inférieure à 0,05 unité ANSON par gramme d'extrait PG et l'extrait P possède une activité principale protéase d'au moins 0,1 unité ANSON par gramme d'extrait P et des activités cellulase et polygalacturonase inférieures à 5 unités par milligramme de protéines.

9. Procédé selon l'une quelconque des revendications 1 à 8 dans lequel les quantités d'extraits employées sont telles que les proportions de chaque extrait, extrait PG et extrait P représentent chacune respectivement en poids de protéine de 0,01 à 10 % par rapport au poids de gomme xanthane.

10. Gomme xanthane en poudre ou en solution aqueuse obtenu par le procédé de l'une quelconque des revendications 1 à 9.

11. Utilisation de la gomme xanthane obtenue par le procédé de l'une quelconque des revendications 1 à 9 comme agent de récupération assistée du pétrole.

**Claims**

1. A process for treating a xanthan gum in order to improve the filterability of its aqueous solutions, comprising an enzymatic treatment of an aqueous solution of xanthan gum whose concentration of dissolved alkali and/or alkaline-earth metal salts is at least $10^{-2}$ equivalent/liter, characterized in that the enzymatic treatment is performed by means of two enzyme extracts of different types, a so-called PG enzyme extract whose main activity is a polygalacturonase activity and a so-called P enzyme extract whose main activity is a protease activity, in conditions compatible with the activity of said enzyme extracts.

2. A process according to claim 1, wherein both enzyme extracts of different types are simultaneously used in such conditions that these extracts of both types are simultaneously active.

3. A process according to claim 1, wherein the enzymatic treatment is performed in two successive steps, first with an enzyme extract of the first type, then with an enzyme extract of the other type, the conditions of each step being so selected that the selected type of extract is active in this step.

4. A process according to claim 3, wherein the first step comprises the treatment with a PG extract and the second step the treatment with a P extract.

5. A process according to claim 4, wherein the first step is conducted with a PG extract at a pH from 3 to 7 and the second step with a P extract at a pH from 6 to 12, these two steps lasting, as a whole, from 0.5 to 60 hours and the temperature being from 15 to 70°C.

6. A process according to any of claims 1 to 5, wherein the PG extract is obtained from the culture of a fungus pertaining to the Aspergillus genus type and the P extract is obtained from the culture of a microorganism of Bacillus type.

7. A process according to one of claims 1 to 6, wherein the PG extract is obtained from a culture of Aspergillus niger.

8. A process according to any one of claims 1 to 7, characterized in that the PG extract has a main polygalacturonase activity and a secondary activity of cellulase type (C) such that the C/PG activities ratio is lower than 1 and the protease activity lower than 0.05 ANSON unit per gram of PG extract, and the P extract has a main protease activity of at least 0.1 ANSON unit per gram of P extract and has cellulase and polygalacturonase activities lower than 5 units per milligram of proteins.

9. A process according to one of claims 1 to 8, wherein the extracts are used in such amounts by weight that the proportion of each of the PG and P extracts respectively represents a protein weight from 0.01 to 10 % of the xanthan gum weight

10. A xanthan gum powder or aqueous solution obtained by the process according to any one of claims 1 to 9.

11. The use of xanthan gum obtained by the process according to any one of claims 1 to 10 as enhanced oil recovery agent.

**Patentansprüche**

1. Verfahren zur Behandlung eines Xanthangummis, um die Filtrierbarkeit seiner wäßrigen Lösungen zu verbessern, das umfaßt eine enzymatische Behandlung einer wäßrigen Xanthangummilösung, die gelöste Alkalimetall- und/oder Erdalkalimetallsalze in einer Konzentration von mindestens $10^{-2}$ Äquivalenten/l enthält, dadurch gekennzeichnet, daß die enzymatische Behandlung durchgeführt wird unter Verwendung von zwei unterschiedlichen enzymatischen Extrakt-Typen, eines enzymatischen Extrakts, als PG-Extrakt bezeichnet, dessen Hauptaktivität eine Polygalacturonase-Aktivität ist, und eines enzymatischen Extrakts, als P-Extakt bezeichnet, dessen Hauptaktivität eine Protease-Aktivität ist, unter Bedingungen, die mit der Aktivität der genannten enzymatischen Extrakte kompatibel sind.

2. Verfahren nach Anspruch 1, bei dem die beiden unterschiedlichen enzymatischen Extrakt-Typen gleichzeitig unter Bedingungen verwendet werden, unter denen diese beiden Extrakt-Typen gleichzeitig aktiv sein können.

3. Verfahren nach Anspruch 1, bei dem die enzymatische Behandlung in zwei aufeinanderfolgenden Stufen durchgeführt wird, zunächst mit einem enzymatischen Extrakt vom ersten Typ, dann mit einem enzymatischen Extrakt vom anderen Typ, wobei die Bedingungen in jeder Stufe so gewählt werden, daß der gewählte Extrakt-Typ während der genannten Stufe aktiv sein kann.

4. Verfahren nach Anspruch 3, bei dem die erste Stufe die Behandlung mit dem PG-Extrakt und die zweite Stufe die Behandlung mit dem P-Extrakt umfaßt.

5. Verfahren nach Anspruch 4, bei dem die erste Stufe mit einem PG-Extrakt bei einem PH-Wert von 3 bis 7 und die zweite Stufe mit einem P-Extrakt bei einem pH-Wert von 6 bis 12 durchgeführt werden, wobei die Gesamtdauer dieser beiden Stufen 0,5 bis 60 stunde beträgt und die Temperatur 15 bis 70°C beträgt.

6. Verfahren nach einem der Ansprüche 1 bis 5, bei dem der PG-Extrakt ein Extrakt ist, der aus einer Kultur eines Pilzes gewonnen wurde, der zum Genus aspergillus gehört, und daß der P-Extrakt aus einer Kultur eines Mikroorganismus vom Typ Bacillus gewonnen wurde.

7. Verfahren nach einem der Ansprüche 1 bis 6, bei dem der PG-Extrakt ein Extrakt ist, der aus einer Aspergillus niger-Kultur gewonnen wurde.

8. Verfahren nach einem der Ansprüche 1 bis 7, dadurch gekennzeichnet, daß der PG-Extrakt eine Polygalacturonase-Aktivität als Hauptaktivität und eine sekundäre Aktivität vom Cellulase (C)-Typ aufweist, so daß das C/PG-Aktivitätsverhältnis unter 1 liegt und die Protease-Aktivität unter 0,05 ANSON-Einheiten pro g PG-Extrakt liegt, und daß der P-Extrakt als Hauptaktivität eine Protease-Aktivität von mindestens 0,1 ANSON-Einheiten pro g P-Extrakt und Cellulase- und Polygalacturonase-Aktivitäten unter 5 Einheiten pro mg Proteine aufweist.

9. Verfahren nach einem der Ansprüche 1 bis 8, bei dem die verwendeten Extrakt-Mengen so sind, daß die Mengenanteile jedes Extrakts, des PG-Extrakts und des P-Extrakts, jeweils 0,01 bis 10 Gew.-% Protein, bezogen auf das Gewicht des Xanthangummis, repräsentieren.

10. Xanthangummi in Pulverform oder in Form einer wäßrigen Lösung, wie es nach dem Verfahren nach einem der Ansprüche 1 bis 9 erhalten wird.

11. Verwendung des nach dem Verfahren nach einem der Ansprüche 1 bis 9 erhaltenen Xanthangummis als Agens zur unterstützten Gewinnung von Erdöl.